# EUROPEAN PATENT APPLICATION

(11) **EP 1 625 824 A1**
(43) Date of publication of application: **15.02.2006**
(21) Application number: 04734099.7
(22) Date of filing: 20.05.2004
(51) Int. Cl.: A61B 5/15

(54) **INSERTION DEPTH-ADJUSTABLE NEEDLE INSERTION DEVICE**

(30) Priority: 21.05.2003 JP 2003143193
(71) Applicant: Arkray, Inc., Kyoto-shi, Kyoto 601-8045 (JP)
(72) Inventor: UEHATA, Yoshiharu, c/o ARKRAY Inc., Kyoto-shi, Kyoto 6018045 (JP); FUKUZAWA, Masahiro, c/o ARKRAY Inc., Kyoto-shi, Kyoto 601845 (JP)
(74) Representative: Pluckrose, Anthony William
(86) International application number: PCT/JP2004/006844
(87) International publication number: WO 2004/103177

(57) **Abstract**

The present invention relates to a lancing depth-adjustable lancing apparatus (1) for moving a lancing element (21) held in a housing (3) in a lancing direction (N1) to lance skin with the lancing element (21). The lancing apparatus (1) includes a lancing depth adjustment mechanism for adjusting lancing depth of the lancing element (21) in the skin. The lancing depth adjustment mechanism includes a displacing member (6) which is movable relative to the housing (3) in the lancing or the retreating direction (N1, N2), a cover (7) which is movable with the displacing member (6) in the lancing or the retreating directions (N1, N2), and a stepped portion (37) provided at the housing (3) for restricting the movement of the lancing element (21) in the lancing direction (N1) when the lancing element (21) moves in the lancing direction (N1).

## Description

### TECHNICAL FIELD

The present invention relates to a lancing apparatus for moving a lancing element to lance skin with the lancing element. Specifically, the present invention relates to a lancing apparatus capable of adjusting the lancing depth of the lancing element in the skin.

### BACKGROUND ART

As an adjustment mechanism for adjusting the lancing depth in a lancing apparatus, a mechanism is known which includes three members, i.e. an inner sleeve, an intermediate ring and an external sleeve, as already disclosed (See Patent Documents 1 and 2, for example).

Specifically, as shown in Figs. 19A and 19B of the present application, in the adjustment mechanism disclosed in Patent Document 1, the intermediate ring 91A is fixed to the inner sleeve 90A, and the outer sleeve 92A moves relative to the intermediate ring 91A in the direction indicated by the arrows N1 and N2 in the figures. The intermediate ring 91A has an inclined end surface 93A on the arrow N1 side. The outer sleeve 92A includes therein an inclined surface 94A for engagement with the end surface 93A of the intermediate ring 91A. With such a structure, by rotating the outer sleeve 92A, the outer sleeve 92A moves relative to the intermediate ring 91A and the inner sleeve 90A in the arrows N1, N2 direction.

As shown in Figs. 20A and 20B of the present application, in the adjustment mechanism disclosed in Patent Document 2, the intermediate ring 91B is fixed to the outer sleeve 92B, and the intermediate ring 91B moves relative to the inner sleeve 90B in the direction indicated by the arrows N1 and N2 in the figures. Specifically, the intermediate ring 91B has an inner surface formed with female threads 95B, whereas the front end of the inner sleeve 90B has an outer surface formed with male threads 96B. With such a structure, by rotating the outer sleeve 92B, the intermediate ring 91B and the outer sleeve 92B move relative to the inner sleeve 90B in the arrows N1, N2 direction.

When the adjustment mechanism 9A, 9B is incorporated in a lancing apparatus, the movement of the lancet L in the arrow N1 direction is restricted by the front end 97A, 97B of the inner sleeve 90A, 90B. Therefore, by moving the outer sleeve 92A, 92B relative to the inner sleeve 90A, 90B in the arrow N1, N2 direction, it is possible to adjust the distance between the front end 97A, 97B of the inner sleeve 90A, 90B and the front end 98A, 98B of the outer sleeve 92A, 92B, and hence, to adjust the length through which the needle L' of the lancet L projects from the outer sleeve 92A, 92B.

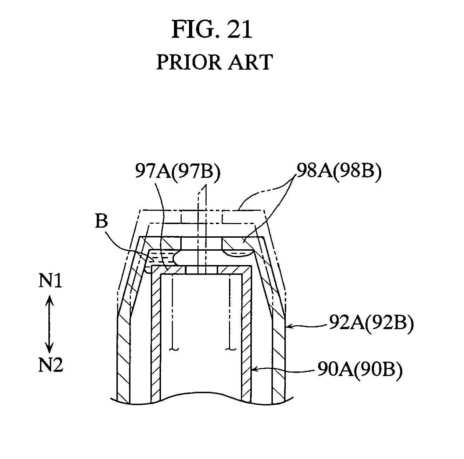

In the adjustment mechanism 9A, 9B, the movement of the lancet L is restricted at the front end 97A, 97B of the inner sleeve 90A, 90B, while the lancing depth is adjusted by adjusting the distance between the front end 98A, 98B of the outer sleeve 92A, 92B and the front end 97A, 97B of the inner sleeve 90A, 90B. Therefore, as shown in Fig. 21, blood B is likely to pool between the front end 98A, 98B of the outer sleeve 92A, 92B and the front end 97A, 97B of the inner sleeve 90A, 90B, which is unsanitary. Therefore, for good hygiene, it is necessary to frequently disassemble and clean the adjustment mechanism 9A, 9B and then assemble the adjustment mechanism 9A, 9B again. However, the adjustment mechanism 9A, 9B is to be attached to or detached from the main body of a lancing apparatus in the assembled state, and the operation for disassembling the adjustment mechanism 9A, 9B is not easy and forces a troublesome work on the user. Further, frequent disassembling and assembling of the adjustment mechanism 9A, 9B increases the risk of breaking the adjustment mechanism 9A, 9B or losing parts such as outer sleeve 92A, 92B. The cleaning of a plurality of complicated parts is troublesome, and there is a high possibility that some portions remain uncleaned. Further, the adjustment mechanism 9A, 9B is sometimes designed not to be disassembled for avoiding breakage or loss of parts. In such a case, the possibility that some portions remain uncleaned increases.
Patent Document 1: JP-A 10-508527
Patent Document 2: WO97/04707

### DISCLOSURE OF THE INVENTION

An object of the present invention is to enable hygienic lancing depth adj ustment stably for a long period of time without requiring complicated maintenance.

According to a first aspect of the present invention, there is provided a lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin; wherein the lancing depth adjustment mechanism comprises a displacing member which is movable relative to the housing in the lancing direction or in a retreating direction which is opposite from the lancing direction, a cover which is movable with the displacing member in the lancing and the retreating directions, and a stepped portion provided at the housing for restricting the movement of the lancing element in the lancing direction when the lancing element moves in the lancing direction.

According to a second aspect of the present invention, there is provided a lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin; wherein the apparatus further comprises a first movable member which is movable in the lancing direction while holding the lancing element, and a second movable member which is connected to the first movable member and which controls movement of the first movable member in accordance with the movement; and wherein the lancing depth adjustment mechanism comprises a displacing member which is movable relative to the housing in the lancing direction or in a retreating direction which is opposite from the lancing direction, and a cover which is movable with the displacing member in the lancing and the retreating directions.

For instance, the first movable member moves reciprocally in the lancing direction and the retreating direction when the second movable member moves in the retreating direction.

According to a third aspect of the present invention, there is provided a lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin; wherein the lancing depth adjustment mechanism comprises a cover which is attachable to and detachable from the housing, and a displacing member which is movable relative to the housing in the lancing direction or in a retreating direction which is opposite from the lancing direction independently from the cover.

For instance, in the lancing apparatus according to the third aspect, the displacing member is provided with a stepped portion for restricting the movement of the lancing element in the lancing direction when the lancing element moves in the lancing direction.

For instance, in the lancing apparatus according to the first through the third aspects of the present invention, the displacing member moves relative to the housing in the lancing or the retreating direction when rotated.

For instance, one of the displacing member and the housing includes a projection, whereas the other one of the displacing member and the housing includes a recess for engagement with the projection. In this case, the lancing depth adjustment mechanism adjusts the position of the displacing member relative to the housing by adjusting engagement relationship between the projection and the recess by rotating the displacing member. Preferably, at least one of the projection and the recess is helical.

For instance, the housing includes a main body, and a guide member non-rotatably fixed to the main body, and preferably, the displacing member is rotatably and threadingly engaged with the guide member. For instance, in the lancing apparatus according to the first embodiment, the stepped portion is provided at the guide member.

For instance, the lancing apparatus according to the present invention includes a fixer for fixing positional relationship between the displacing member and the housing. Preferably, the fixer includes one or more projections formed at one of the displacing member and the housing, and one or more recesses formed at the other one of the displacing member and the housing for engagement with the projections. Preferably, either of said one or more projections and said one or more recesses is capable of displacing in a direction crossing the lancing direction.

For instance, there are at least a plurality of the recesses or a plurality of the projections. In this case, the plurality of projections or recesses are aligned in a rotational direction of the displacing member.

According to a fourth aspect of the present invention, there is provided a lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin; wherein the lancing depth adjustment mechanism utilizes a cam mechanism and comprises a displacing member which is movable relative to the housing in the lancing direction or in a retreating direction which is opposite from the lancing direction, and a cover which is movable with the displacing member in the lancing and the retreating directions.

For instance, the housing is provided with a stepped portion for restricting the movement of the lancing element in the lancing direction when the lancing element moves in the lancing direction.

For instance, the cam mechanism comprises an operation member which is used to move the displacing member and which includes a shaft portion, and a first and a second groove portions respectively provided at the housing and the displacing member for allowing movement of the shaft portion. Preferably, in this case, the lancing depth adjustment mechanism changes the position of the displacing member relative to the housing by moving the shaft portion in the first and the second groove portions.

For instance, one of the first and the second groove portions extends in a direction perpendicular to the lancing and the retreating directions, whereas the other one of the first and the second groove portions extends in a direction inclined with respect to the lancing and the retreating directions.

Preferably, in the lancing apparatus according to the first through the fourth aspects of the present invention, the cover is attachable to and detachable from the displacing member or the housing, and the position of the displacing member relative to the housing is adjusted by applying a load to the displacing member with the cover detached. For instance, the cover covers at least part of the displacing member when attached to the displacing member. Preferably, in this case, the part of the displacing member to be covered by the cover is provided with a scale used for adjusting the lancing depth.

For instance, the cover is made transparent or translucent so that inside can be observed. In the structure in which the cover is attached to the displacing member, the cover may be made rotatable independently from the displacing member.

### BRIEF DESCRIPTION OF THE DRAWINGS

Fig. 1 is an entire perspective view showing a lancing apparatus according to a first embodiment of the present invention.
Fig. 2 is a perspective view showing the state in which the cover is detached from the state shown in Fig. 1.
Fig. 3 is a sectional view taken along lines III-III in Fig. 1.
Fig. 4 is an exploded perspective view of a front end of the lancing apparatus shown in Fig. 1.
Fig. 5 is a sectional view of a guide member of the lancing apparatus shown in Fig. 1.
Fig. 6A is an entire perspective view of a rotatable member of the lancing apparatus shown in Fig. 1, and Fig. 6B is a sectional view thereof.
Fig. 7 is a sectional view for describing the operation for mounting a lancet to the lancing apparatus shown in Fig. 1.
Fig. 8 is a sectional view for describing the operation for mounting a lancet to the lancing apparatus shown in Fig. 1.
Figs. 9A and 9B are sectional views for describing the operation for adjusting the lancing depth in the lancing apparatus shown in Fig. 1.
Fig. 10 is a sectional view for describing the lancing operation of the lancing apparatus shown in Fig. 1.
Fig. 11 is a sectional view for describing the lancing operation of the lancing apparatus shown in Fig. 1.
Fig. 12 is a sectional view of a lancing apparatus according to a second embodiment of the present invention.
Fig. 13 is a schematic view for describing the lancing operation of the lancing apparatus shown in Fig. 12.
Fig. 14 is a sectional view showing a principal portion of a lancing apparatus according to a third embodiment of the present invention.
Fig. 15 is an entire perspective view showing a lancing apparatus according to a fourth embodiment of the present invention.
Fig. 16 is a sectional view taken along lines XVI-XVI in Fig. 15.
Fig. 17 is a sectional view taken along lines XVII-XVII in Fig. 15.
Figs. 18 are front views, partially cut away, for describing the lancing operation of the lancing apparatus shown in Fig. 15.

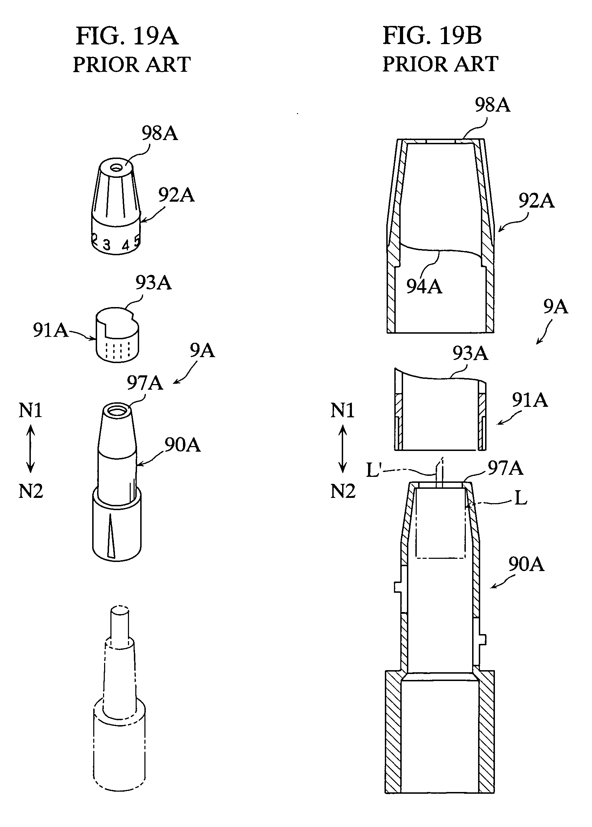

Fig. 19A is an exploded perspective view for describing an example of lancing depth adjustment mechanism in a prior art lancing apparatus, and Fig. 19B is an exploded sectional view thereof.

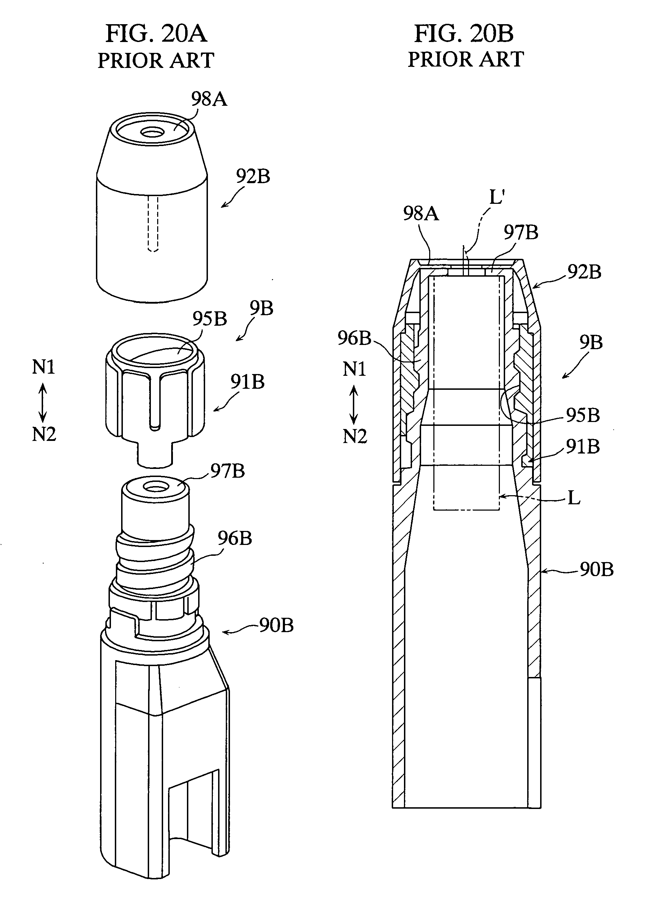

Fig. 20A is an exploded perspective view for describing another example of lancing depth adjustment mechanism in a prior art lancing apparatus, and Fig. 20B is a sectional thereof in an assembled state.
Fig. 21 is a sectional view showing a principal portion of an adjustment mechanism for describing problems of a prior art adjustment mechanism.

### BEST MODE FOR CARRYING OUT THE INVENTION

Preferred embodiments of the present invention will be described below as a first through a fourth embodiments with reference to the accompanying drawings.

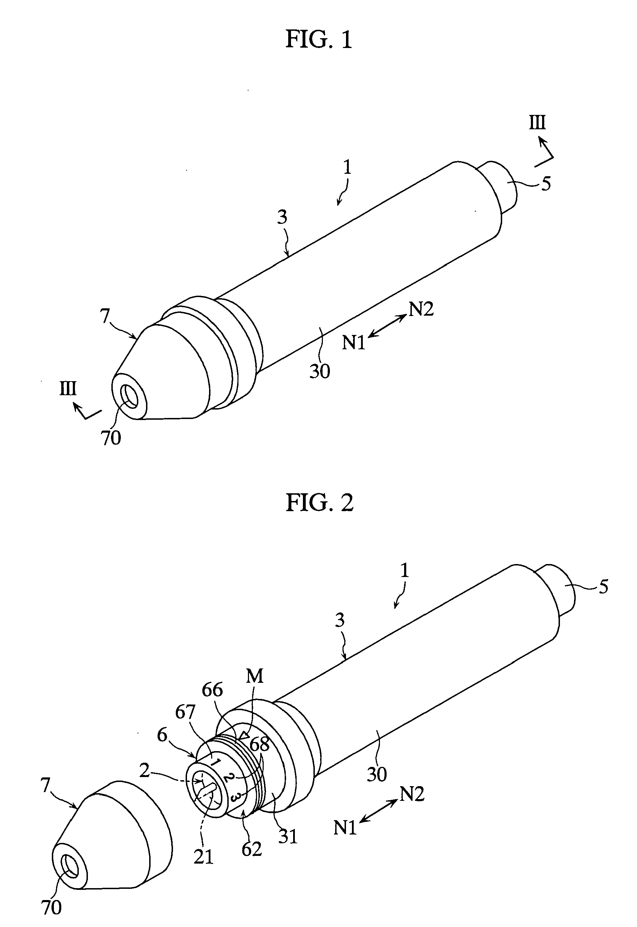

First, with reference to Figs. 1 through 11, a first embodiment of the present invention will be described.

The lancing apparatus 1 shown in Figs. 1-3 is used with a lancet 2 mounted thereto. The lancet 2 includes a main body 20, and a needle 21 projecting from the main body. For example, in molding the main body 20 using synthetic resin, the needle 21 is integrally formed on the main body 20 by insert molding.

The lancing apparatus 1 includes a housing 3, a lancet holder 4, an operation cap 5, a rotatable member 6 and a cover 7.

The housing 3 accommodates the lancet holder 4. The housing 3 includes a main body 30, and a guide member 31 fixed to the main body.

The main body 30 includes a through-hole 32, a projection 33 and an annular recess 34. The through-hole 32, which allows the movement of the operation cap 5, is provided at an upper wall 35 of the housing 3. The projection 33 serves to hold coil springs S1 and S2 and engage with the lancet holder 4. The annular recess 34 serves to fix the guide member 31 to the main body 30.

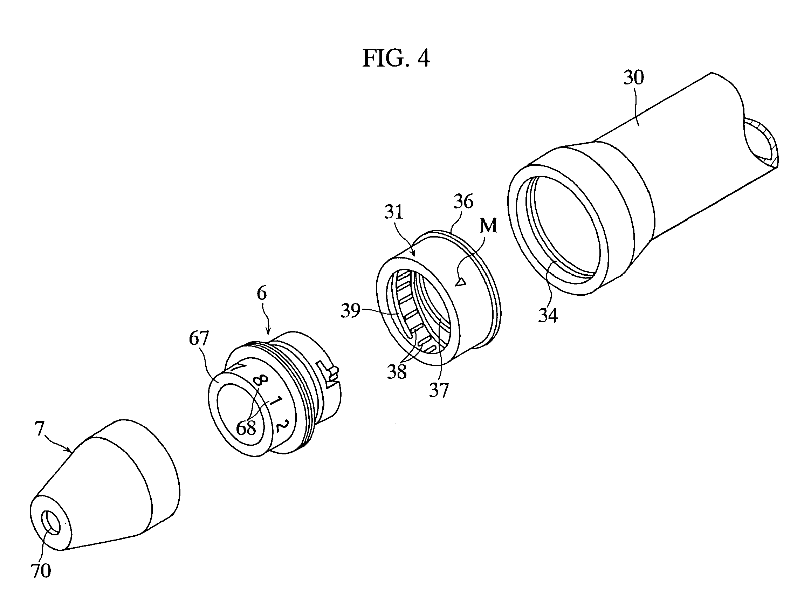

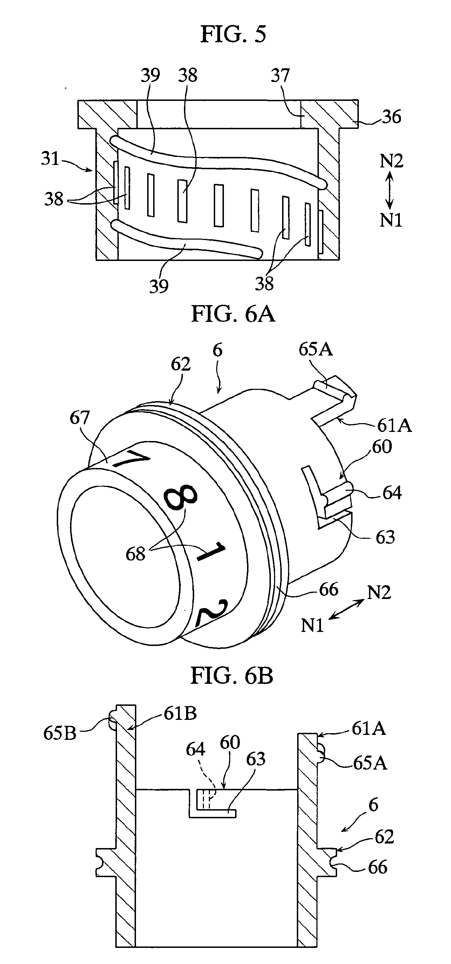

The guide member 31 serves to restrict the movement of the lancet holder 4 in the lancing direction N1, which will be described later, and to support the rotatable member 6. As better shown in Figs. 4 and 5, the guide member 31 includes an outer flange portion 36, an inner flange portion 37, a plurality of linear recesses 38, and a helical groove 39.

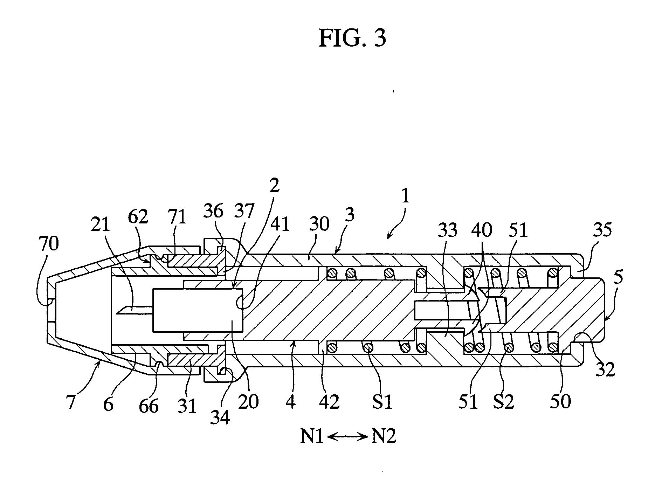

The outer flange portion 36 is utilized for fixing the guide member 31 to the main body 30 and fitted in the annular recess 34 of the main body 30. By the fitting, the guide member 31 is non-rotatably fixed to the main body 30. The inner flange portion 37 serves to engage with a flange portion 42 of the lancet holder 4, which will be described later (See Fig. 3). The linear recesses 38 serve to engage with a projection 64 (See Fig. 6A) of a movable portion 60 of the rotatable member 6, which will be described later. The recesses extend in the lancing and the retreating directions N1 and N2, and are regularly spaced in the circumferential direction of the inner surface of the guide member 31. The helical groove 39 serves to engage with inclined projections 65A and 65B (See Figs. 6A and 6B) of projecting pieces 61, 61B of the rotatable member 6, which will be described later.

As will be understood from Fig. 3, the lancet holder 4 serves to hold the lancet 2 and is movable in the lancing direction N1 by pushing the operation cap 5. The lancet holder 4 is provided with a pair of engagement claws 40, a recess 41 and a flange portion 42.

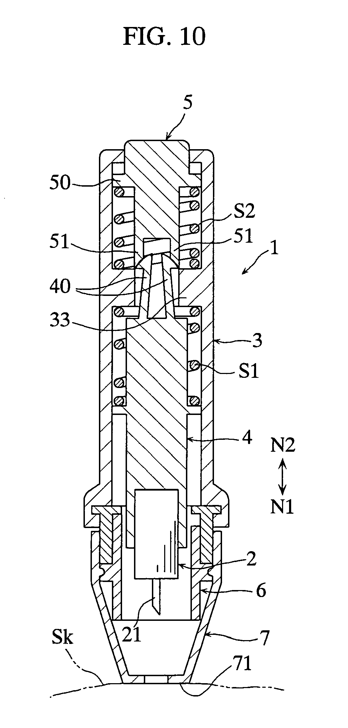

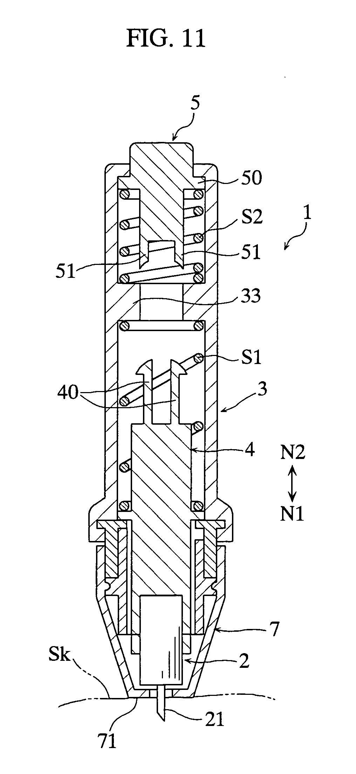

The paired engagement claws 40 are engageable with the projection 33 of the housing 3 and made resilient to be movable toward and away from each other (See Fig. 10). The recess 41 serves to hold the lancet 2. The flange portion 42 serves to engage the inner flange portion 37 of the guide member 31 as noted above and hold the coil spring S1 in cooperation with the projection 33 of the housing 3. When the paired engagement claws 40 are brought into engagement with the projection 33, the coil spring S1 is compressed. Therefore, when the paired engagement claws 40 are disengaged from the projection 33, the lancet holder 4 moves in the lancing direction N1 due to the resilient force of the coil spring S1 (See Figs. 10 and 11).

The operation cap 5, which is utilized for moving the lancet holder 4 in the lancing direction N1, is slidably held by the housing 3, with a portion thereof projecting from the through-hole 32 of the housing 3. The operation cap 5 includes a flange portion 50 and a pair of push portions 51. The coil spring S2 is arranged between the flange portion 50 and the projection 33 of the housing 3. The flange portion 50 in the natural state is held in engagement with the upper wall 35 of the housing 3. When the operation cap 5 is pushed in the lancing direction N1, the operation cap 5 moves in the lancing direction N1 while compressing the coil spring S2 (See Fig. 10). When the pressing force exerted on the operation cap 5 is removed, the operation cap 5 returns to its original position due to the resilient force of the coil spring S2 (See Fig. 11).

When the operation cap 5 is moved in the lancing direction N1 more than a predetermined distance as shown in Fig. 10, the push portions 51 push the paired engagement claws 40 to move the engagement claws 40 so that the respective ends thereof come close to each other. By the action of the push portions 50, the paired engagement claws 40 are released from the engagement state (latched state) with the projection 33. As a result, as noted above, the lancet holder 4 moves in the lancing direction N1 due to the resilient force of the coil spring S1 (See Fig. 11).

As shown in Figs. 6A and 6B, the rotatable member 6, which is operated in adjusting the lancing depth, includes a movable portion 60, a pair of projecting pieces 61A, 61B and a flange portion 62.

The movable portion 60 is formed with an L-shaped cutout 63 to have spring properties, and includes a projection 64 extending in the arrows N1, N2 direction. As noted above, the projection 64 serves to engage the linear recesses 38 (See Fig. 5) of the guide member 31. The paired projecting pieces 61A and 61B project in the arrow N2 direction while facing each other across the center of the rotatable member 6 and differ from each other in height. The projecting pieces 61A and 61B are formed with inclined projections 65A and 65B, respectively. As noted above, the inclined projections 65A and 65B serve to engage the helical groove 39 of the guide member 31 and are arranged on a helical track of the same pitch as that of the helical groove 39 (See Fig. 5). The number of projecting pieces of the rotatable member 6 is not limited to two and may be one or no less than three. The flange portion 62 is utilized for mounting the cover 7 and includes an annular recess 66 for engaging the annular projection 71 of the cover 7, which will be described later (See Fig. 3).

The rotatable member 6 includes an end 67 provided with a scale 68. The scale 68 is utilized for adjusting the lancing depth. Specifically, as shown in Figs. 2 and 4, the lancing depth can be adjusted by rotating the rotatable member 6 and aligning a selected number in the scale 68 with a mark M provided at the outer surface of the guide member 31.

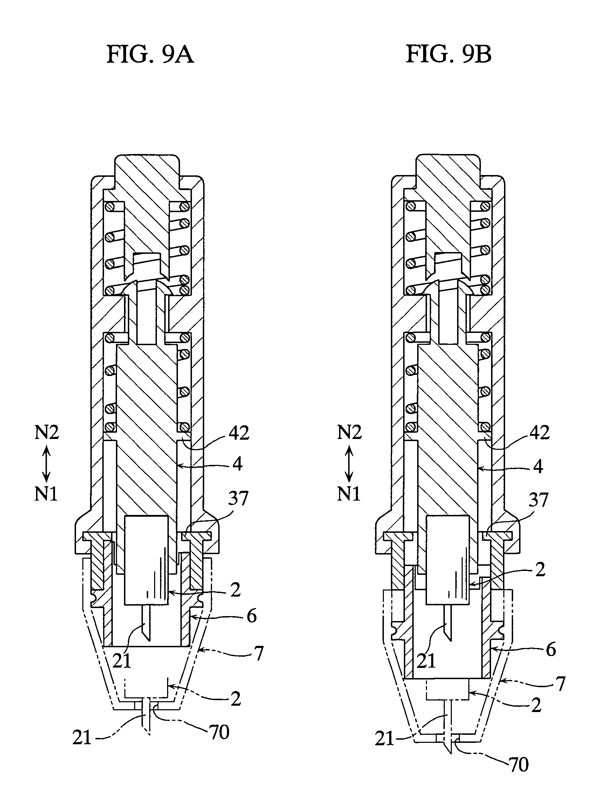

As will be perceived from Figs. 4-6B, when the rotatable member 6 is rotated with the projection 64 and the inclined projections 65A and 65B held in engagement with the linear recess 38 and the helical groove 39 of the guide member 31, the inclined projections 65A and 65B move through the helical groove 39 to perform helical movement. As a result, the rotatable member 6 moves relative to the guide member 31 in the lancing or the retreating direction N1, N2 (See Figs. 9A and 9B). In accordance with the rotation of the rotatable member 6, the projection 64 also performs helical movement similarly to the inclined projections 65A and 65B. During this movement, the projection successively engages one of the linear recesses 38 correspondingly to the positions of the inclined projections 65A, 65B. Therefore, when the rotatable member 6 is rotated, click feeling is obtained due to the engagement of the projection 64 and the linear recesses 38. Further, by stopping the rotation of the rotatable member 6 with the projection 64 engaging the linear recess 38, the rotatable member 6 is kept fixed to the guide member 31.

The cover 7 shown in Figs. 1-4 is brought into contact with skin in sticking the needle 21 of the lancet 2 into the skin. The cover 7 is made entirely transparent so that the inside of the cover can be observed. Therefore, even when the cover 7 is mounted to the rotatable member 6, whether or not the lancet 2 is mounted to the lancet holder 4 can be checked, whereby erroneous operation is prevented. Further, after the lancing operation, whether or not blood has entered the cover 7 can be checked. Therefore, it is sufficient to clean the cover 7 only when blood has entered the cover 7, and the cleaning need not be performed after each time of lancing operation. Therefore, the troublesome work for maintenance is reduced.

The cover 7 has a front end formed with a through-hole 70 and an inner surface formed with an annular projection 71. The through-hole 70 allows the needle 21 of the lancet 2 to project. The annular projection 71 serves to engage the annular recess 66 of the flange portion 62 of the rotatable member 6, as noted above. Though not clearly shown in the figure, the annular projection 71 is made smaller in width and height than the annular recess 66 of the flange portion 62 of the rotatable member 6. Therefore, when the cover 7 is rotated, the cover 7 rotates independently from the rotatable member 6, and the cover 7 is detachable from the rotatable member 6. Firstly, therefore, even when any external force is exerted on the cover 7 or the user rotates the cover 7, the rotatable member 6 does not rotate, so that the lancing depth once set is not changed unintentionally. Secondly, even when blood enters the cover 7, the cover can be easily and reliably cleaned by detaching the cover 7. Therefore, the troublesome work for maintenance is reduced, and good hygiene is provided. Further, since the interior of the cover 7 can be cleaned with the cover 7 detached, the entirety of the mechanism for adjusting the lancing depth need not be disassembled. Also from this point, the burden of maintenance can be reduced.

As noted above, the cover 7 is mounted to the rotatable member 6 by bringing the annular projection 71 formed at the inner surface of the cover into engagement with the flange portion 62 of the rotatable member 6. Therefore, with the cover 7 mounted to the rotatable member 6, the end 67 of the rotatable member 6 provided with the scale 68 is accommodated in the cover 7. In this way, with the cover 7 mounted to the rotatable member 6, the scale 68 is not exposed and does not come into view, so that the user is freed from the obsession or impulse to operate the rotatable member 6. Therefore, the impression that the operation of the lancing apparatus 1 is complicated is reduced.

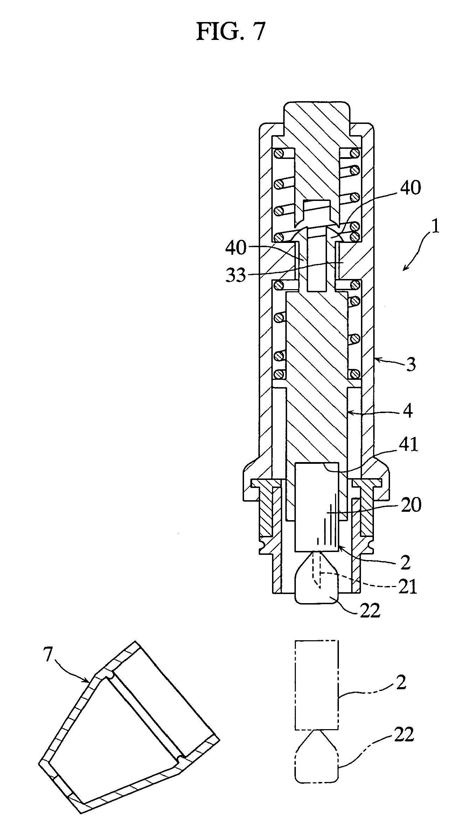

The usage of the lancing apparatus 1 will be described below. In the lancing operation, the lancet 2 is first mounted to the lancet holder 4, as shown in Fig. 3. As shown in Fig. 7, to mount the lancet 2, with the cover 7 detached from the housing 3, the main body 20 of the lancet 2 provided with a protective cap 22 is fitted into the recess 41 of the lancet holder 4 from the side opposite from the needle 21. At this time, the paired engagement claws 40 of the lancet holder 4 may be brought into engagement with the projection 33 of the housing 3 to provide the latched state. Alternatively, the latching may be performed separately from the mounting operation of the lancet 2. That is, the latched state may be provided before the lancet 2 is mounted.

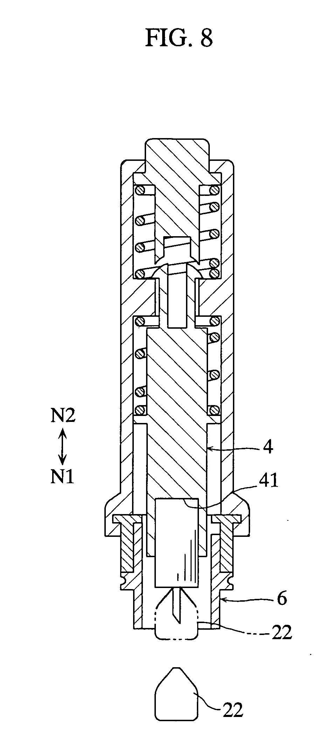

As shown in Fig. 8, after the lancet 2 with the protective cap 22 is mounted to the lancet holder 4, the protective cap 22 is removed. The removal of the protective cap 22 may be performed by holding the protective cap 22 with fingers and cutting away the protective cap 22 by twisting.

Before or after the mounting of the lancet 2, the lancing depth is adjusted as required. The adjustment of the lancing depth is performed by rotating the rotatable member 6 to move the rotatable member 6 relative to the housing 3 in the lancing or the retreating direction N1, N2. As noted above, the adjustment of the lancing depth is performed by aligning a selected number in the scale 68 with the mark M of the guide member 31. As indicated by phantom lines in Figs. 9A and 9B, since the cover 7 is later mounted to the rotatable member 6, the position of the cover 7 in lancing and hence the position of the through-hole 70 of the cover 7 can be selected by moving the rotatable member 6 in the lancing or the retreating direction N1, N2. It is to be noted that the movement of the lancet holder 4 in the lancing direction N1 is stopped by the engagement of the flange portion 42 with the inner flange portion 37 of the guide member 31, and the position of the inner flange portion 37 is fixed and does not change due to the rotation of the rotatable member 6. Therefore, the position where the lancet holder 4 is stopped is constant regardless of the position of the rotatable member 6. Therefore, by changing the position of the cover 7 (through-hole 70) by rotating the rotatable member 6, the projecting amount of the needle 21 of the lancet 2 from the through-hole 70 can be adjusted.

Subsequently, as shown in Fig. 10, with the cover 7 mounted to the rotatable member 6, the end surface 71 of the cover 7 is brought into close contact with the skin Sk. Thereafter, as shown in Fig. 11, the lancet 2 is moved in the lancing direction N1 to stick the needle 21 of the lancet 2 into the skin Sk. As shown in Fig. 10, the sticking of the needle 21 into the skin Sk is performed by pushing down the operation cap 5 in the lancing direction N1 to release the latched state of the lancet holder 4. As a result, as shown in Fig. 11, the lancet holder 4 moves in the lancing direction N1 due to the resilient force of the coil spring S1, whereby the needle 21 of the lancet 2 sticks into the skin Sk. Thus, the skin Sk is cut and bleeds. Since the coil spring S1 is fixed to the projection 33 of the housing 3 and the flange portion 42 of the lancet holder 4, the coil spring S1 is expanded when the needle 21 sticks in the skin Sk. Therefore, the needle 21 is immediately pulled out from the skin Sk due to the resilient force of the coil spring S1. Therefore, the time period during which the needle 21 sticks in the skin Sk is short, whereby the pain by the lancing can be reduced.

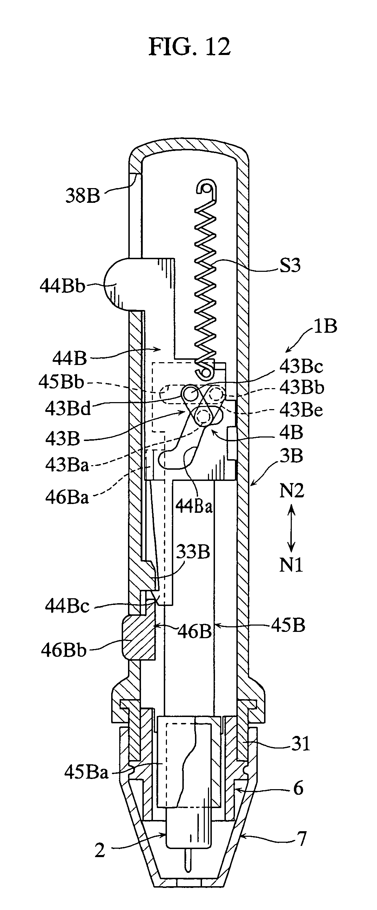

A second embodiment of the present invention will be described below with reference to Figs. 12-13C. In the figures to be referred to below, the elements which are identical or similar to those of the lancing apparatus of the first embodiment are designated by the same reference signs as those used for the first embodiment.

The lancing apparatus 1B shown in Fig. 12 includes a lancing depth adjustment mechanism similar to that of the lancing apparatus 1 (See Fig. 3, for example) of the first embodiment. Specifically, in the lancing depth adjustment mechanism, the cover 7 is attached to the rotatable member 6 for integral movement, and the rotatable member 6 is threadingly engaged with the guide member 3. Therefore, also with the lancing apparatus 1B, the same advantages as those of the above-described lancing apparatus 1 (See Fig. 3, for example) can be obtained.

However, the lancing apparatus 1B differs from the lancing apparatus 1 (See Fig. 3, for example) in structure for moving the lancet holder 45. Specifically, in the lancing apparatus 1B, the lancet 2 is moved by utilizing a cam mechanism 4B, and the movement of the lancet 2 in the lancing direction N1 is restricted without causing engagement with the housing 3B.

The cam mechanism 4B includes a link member 43B, a movable plate 44B and a lancet holder 45B, and is designed to convert the reciprocal movement of the movable plate 44B into the reciprocal movement of the lancet holder 45B via the circular motion of the link member 43B.

The link member 43B connects the movable plate 44B and the lancet holder 45B to each other, and when the movable plate 44B moves, the link member moves the lancet holder 45B in accordance with the movement of the movable plate. The link member 43B includes a first movable pin 43Ba, a second movable pin 43Bb, and a fixed pin 43Bc. The pins 43Ba, 43Bb and 43Bc are connected to each other via armmembers 43Bd and 43Be. Though not clearly shown in the figures, the fixed pin 43Bc is fixed to the housing 3B. With such an arrangement, the link member 43B is rotatable around the fixed pin 43Bc relative to the housing 3B.

The movable plate 44B is movable relative to the housing 3B in the lancing and the retreating directions N1, N2 and connected to the housing 3B via a coil spring S3. The movable plate 44B includes a groove 44Ba, an operation portion 44Bb and a hook portion 44Bc. The groove 44Ba allows the movement of the first movable pin 43Ba of the link member 43B. The operation portion 44Bb, which is used in manually moving the movable plate 44B, is movable in the lancing and the retreating directions N1, N2 due to the provision of an opening 38B in the housing 3B. The hook portion 44Bc serves to engage the projection 33B of the housing 3B to latch the movable plate 44B to the housing 3B. When the hook portion 44Bc is brought into engagement with the projection 33B, the coil spring S3 is expanded, and the movable plate 44B is biased in the retreating direction N2. Therefore, when the hook portion 44Bc is disengaged from the projection 33B, the movable plate 44B moves in the retreating direction N2 due to the resilient force of the coil spring S3. The disengagement of the hook portion 44Bc is performed by utilizing a latch release member 46B. The latch release member 46B, which has appropriate resiliency, includes a fixed end 46Ba fixed to the housing 3B and a free end 46Bb pivotable around the fixed end 46Ba. The free end 46Bb is arranged adj acent the hook portion 44Bc so as to come into contact with the hook portion 44Bc when pressed.

The lancet holder 45B serves to hold the lancet 2 and move the lancet 2. Similarly to the movable plate 44B, the lancet holder is movable in the lancing and the retreating directions N1, N2. The lancet holder 45B includes a holder portion 45Ba for holding the lancet 2, and a groove 45Bb for connection to the movable plate 44B via the link member 43B. The groove 45Bb allows the movement of the second movable pin 43Bb of the link member 43B and extends in a direction perpendicular to the lancing and the retreating directions N1, N2.

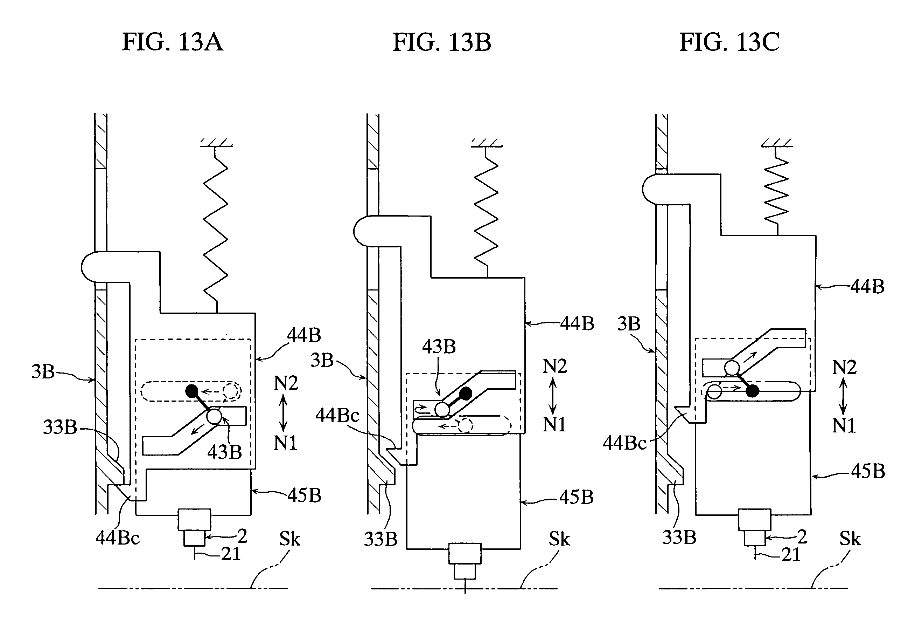

In the lancing apparatus 1B, as shown in Figs. 12-13C, the needle 21 of the lancet 2 can be caused to stick into the skin Sk by pressing the free end 46Bb of the latch release mechanism 46B after the hook portion 44Bc of the movable plate 44B is brought into engagement with the projection 33B. When the free end 46Bb of the latch release member 46B is pressed, the free ends 46Bb acts on the hook portion 44Bc to disengage the hook portion 44Bc from the projection 33B. As a result, as shown in Figs. 13A and 13B, the movable plate 44B moves in the retreating direction N2. In accordance with the movement of the movable plate, the link member 43B rotates clockwise to push down the lancet holder 45B in the lancing direction N1, whereby the needle 21 of the lancet 2 sticks into the skin Sk. Thereafter, as shown in Figs. 13B and 13C, the movable plate 44B further moves in the retreating direction N2, and in accordance with the movement, the lancet holder 45B is lifted in the retreating direction N2, whereby the needle 21 of the lancet 2 is pulled out from the skin Sk.

The cam mechanism of this embodiment is merely an example, and the movement of the lancet holder in the lancing and the retreating directions may be caused by utilizing other cam mechanisms. For example, a member corresponding to the movable plate may be caused to perform circular motion in the housing, and the lancet holder may be moved in the lancing or the retreating direction in accordance with the circular motion.

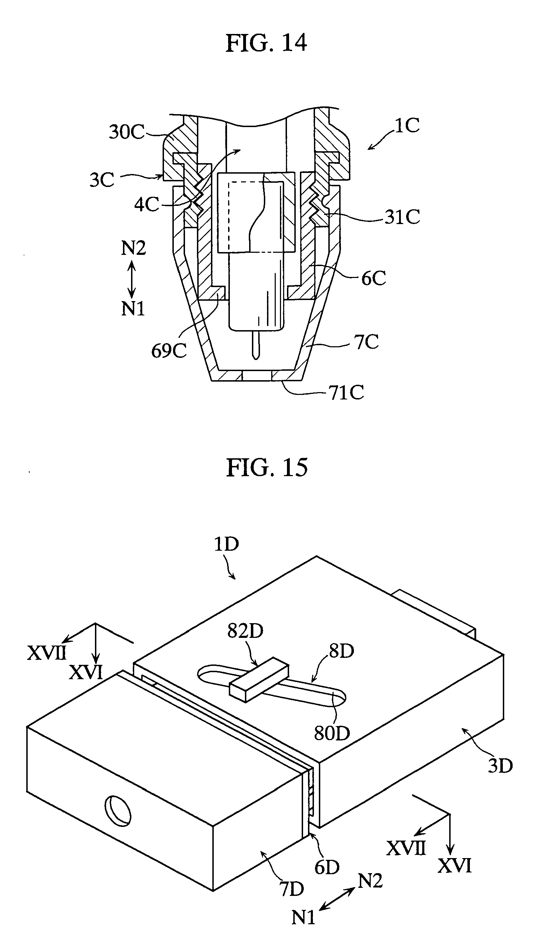

A lancing apparatus according to a third embodiment of the present invention will be described below with reference to Fig. 14.

The lancing apparatus 1C shown in Fig. 14 includes a lancing depth adjustment mechanism which is different from that of the first embodiment. The lancing depth adjustment mechanism includes a guide member 31C, a rotatable member 6C and a cover 7C.

The guide member 31C is non-rotatably fixed to the main body 30C of the housing 3C. The rotatable member 6C is threadingly engaged with the guide member 31C and is movable in the lancing or the retreating direction N1, N2 when rotated around the lancing or the retreating direction N1, N2. The rotatable member 6C includes a flange portion 69C for engaging the lancet holder 4C. The cover 7C is attached to the guide member 31C rotatably but so as not to move in the lancing and the retreating directions N1, N2 and is detachable from the guide member 31C.

With the above arrangement, by rotating the rotatable member 6C, the distance between the end surface 71C of the cover 7C and the flange portion 69C of the rotatable member 6C can be changed. Therefore, the lancing depth can be adjusted by adjusting the position of the flange portion 69C.

In the lancing apparatus 1C again, the cover 7C is attached to the guide member 31C rotatably but so as not to move in the lancing and the retreating directions N1, N2 and detachable from the guide member 31C. Firstly, therefore, even when any external force is exerted on the cover 7C or the user rotates the cover 7C, the rotatable member 6C does not rotate, so that the lancing depth once set is not changed unintentionally. Secondly, even when blood enters the cover 7C, the cover can be easily and reliably cleaned by detaching the cover 7C. Therefore, the troublesome work for maintenance is reduced, and good hygiene is provided. Further, since the interior of the cover 7C can be cleaned with the cover 7C detached, the entirety of the mechanism for adjusting the lancing depth need not be disassembled. Also from this point, the burden of maintenance can be reduced.

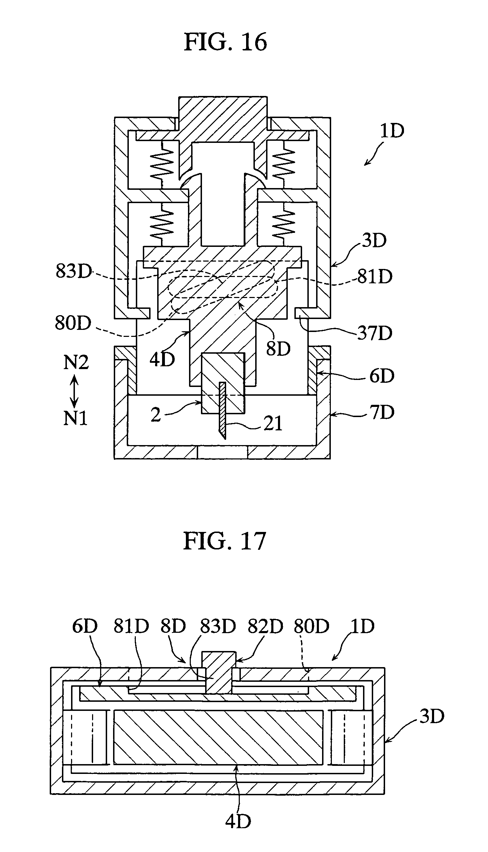

Next, a lancing apparatus according to a fourth embodiment of the present invention will be described below with reference to Figs. 15-18C.

The lancing apparatus 1D shown in Figs. 15-17 is designed to adjust the lancing depth by utilizing a cam mechanism 8D and includes a housing 3D, a lancet holder 4D, a displacing member 6D and a cover 7D.

The cam mechanism 8D includes a first groove 80D, a second groove 81D and an operation member 82D. The first groove 80D penetrates through the housing 3D and is inclined with respect to the lancing and the retreating directions N1, N2. The second groove 81D is formed at the displacing member 6D so as not to penetrate therethrough and extends perpendicularly to the lancing and the retreating directions N1, N2. The operation member 82D includes a shaft portion 83D which is movable through the first and the second grooves 80D and 81D.

Although the configurations of the housing 3D, the lancet holder 4D and the cover 7D differ from those of the first embodiment, the functions are basically the same. Specifically, the lancet holder 4D is movable within the housing 3D, and the needle 21 of the lancet 2 sticks into the skin as a result of the movement of the lancet holder 4D in the lancing direction N1. The movement of the lancet holder 4D in the lancing direction N1 is stopped by the engagement with the flange portion 37D of the housing 3D. As noted above, however, the first groove 80D, which is a structural element of the cam mechanism 8D, is provided at the housing 3D, which is the difference from the first embodiment.

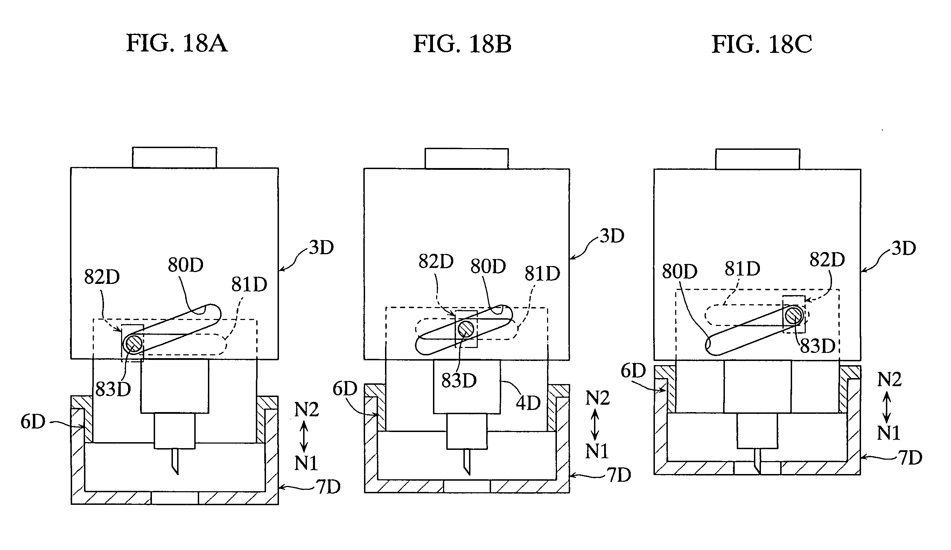

As shown in Figs. 18A-18C, in the lancing apparatus 1D, the lancing depth is adjusted by moving the operation member 82D in a direction perpendicular to the lancing and the retreating directions N1, N2. Specifically, when the operation member 82D is moved, the shaft portion 83D moves straight in the first and the second groove portions 80D and 81D. At this time, since the first groove 80D is inclined, the shaft portion 83D moves through the second groove 81D while moving the displacing member 6D in the lancing or the retreating direction N1, N2. Since the cover 7D moves together with the displacing member 6D, the position of the cover 7D relative to the housing 3D changes in accordance with the movement of the displacing member 6D. Therefore, by moving the operation member 82D to move the displacing member 6D, the cover 7D is moved, whereby the lancing depth can be adjusted.

The cam mechanism 8D of this embodiment is merely an example, and the cam mechanism for adjusting the lancing depth is not limited to the above-described ones.

The present invention is not limited to the foregoing embodiments and may be modified in various ways. For example, the guide member may be eliminated, and the housing may consist of a single member. The mechanism for moving the lancet is not limited to the one utilizing the force of a spring, and those utilizing a force of air or an electromagnetic force or other known mechanisms may be employed.

## Claims

1. A lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin;
wherein the lancing depth adjustment mechanism comprises a displacing member which is movable relative to the housing in the lancing direction or in a retreating direction which is opposite from the lancing direction, a cover which is movable with the displacing member in the lancing and the retreating directions, and a stepped portion provided at the housing for restricting the movement of the lancing element in the lancing direction when the lancing element moves in the lancing direction.

2. The lancing depth-adjustable lancing apparatus according to claim 1, wherein the displacing member moves relative to the housing in the lancing or the retreating direction when rotated.

3. The lancing depth-adjustable lancing apparatus according to claim 2, wherein one of the displacing member and the housing includes a projection, whereas the other one of the displacing member and the housing includes a recess for engagement with the projection; and
wherein the lancing depth adjustment mechanism adjusts a position of the displacing member relative to the housing by adjusting engagement relationship between the projection and the recess by rotating the displacing member.

4. The lancing depth-adjustable lancing apparatus according to claim 3, wherein at least one of the projection and the recess is helical.

5. The lancing depth-adjustable lancing apparatus according to claim 2, wherein the housing includes a main body, and a guide member non-rotatably fixed to the main body; and
wherein the displacing member is rotatably and threadingly engaged with the guide member.

6. The lancing depth-adjustable lancing apparatus according to claim 5, wherein the stepped portion is provided at the guide member.

7. The lancing depth-adjustable lancing apparatus according to claim 1, further comprising a fixer for fixing positional relationship between the displacing member and the housing.

8. The lancing depth-adjustable lancing apparatus according to claim 7, wherein the fixer includes one or more projections formed at one of the displacing member and the housing, and one or more recesses formed at the other one of the displacing member and the housing for engagement with the projections.

9. The lancing depth-adjustable lancing apparatus according to claim 8, wherein either of said one or more projections and said one or more recesses is capable of displacing in a direction crossing the lancing direction.

10. The lancing depth-adjustable lancing apparatus according to claim 2, further comprising a fixer for fixing positional relationship between the displacing member and the housing, the fixer including one or more projections formed at one of the displacing member and the housing, and one or more recesses formed at the other one of the displacing member and the housing for engagement with the projections;
wherein there are at least a plurality of the recesses or a plurality of the projections; and
wherein the plurality of projections or recesses are aligned in a rotational direction of the displacing member.

11. The lancing depth-adjustable lancing apparatus according to claim 1, wherein the cover is attachable to and detachable from the displacing member, and a position of the displacing member relative to the housing is adjusted by applying a load to the displacing member with the cover detached.

12. The lancing depth-adjustable lancing apparatus according to claim 11, wherein the cover covers at least part of the displacing member when attached to the displacing member; and
wherein the part of the displacing member to be covered by the cover is provided with a scale used for adjusting the lancing depth.

13. The lancing depth-adjustable lancing apparatus according to claim 1, wherein the cover is made transparent or translucent so that inside can be observed.

14. The lancing depth-adjustable lancing apparatus according to claim 2, wherein the cover is rotatable independently from the displacing member.

15. A lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin;
wherein the apparatus further comprises a first movable member which is movable in the lancing direction while holding the lancing element, and a second movable member which is connected to the first movable member and which controls movement of the first movable member in accordance with the movement; and
wherein the lancing depth adjustment mechanism comprises a displacing member which is movable relative to the housing in the lancing direction or in a retreating direction which is opposite from the lancing direction, and a cover which is movable with the displacing member in the lancing and the retreating directions.

16. The lancing depth-adjustable lancing apparatus according to claim 15, wherein the first movable member moves reciprocally in the lancing direction and the retreating direction when the second movable member moves in the retreating direction.

17. The lancing depth-adjustable lancing apparatus according to claim 15, wherein the displacing member moves relative to the housing in the lancing or the retreating direction when rotated.

18. The lancing depth-adjustable lancing apparatus according to claim 17, wherein one of the displacing member and the housing includes a projection, whereas the other one of the displacing member and the housing includes a recess for engagement with the projection; and
wherein the lancing depth adjustment mechanism adjusts a position of the displacing member relative to the housing by adjusting the engagement relationship between the projection and the recess by rotating the displacing member.

19. The lancing depth-adjustable lancing apparatus according to claim 18, wherein at least one of the projection and the recess is helical.

20. The lancing depth-adjustable lancing apparatus according to claim 19, wherein the housing includes a main body, and a guide member non-rotatably fixed to the main body; and
wherein the displacing member is rotatably and threadingly engaged with the guide member.

21. The lancing depth-adjustable lancing apparatus according to claim 15, further comprising a fixer for fixing positional relationship between the displacing member and the housing.

22. The lancing depth-adjustable lancing apparatus according to claim 21, wherein the fixer includes one or more projections formed at one of the displacing member and the housing, and one or more recesses formed at the other one of the displacing member and the housing for engagement with the projections.

23. The lancing depth-adjustable lancing apparatus according to claim 22, wherein either of said one or more projections and said one or more recesses is capable of displacing in a direction crossing the lancing direction.

24. The lancing depth-adjustable lancing apparatus according to claim 17, further comprising a fixer for fixing positional relationship between the displacing member and the housing, the fixer including one or more projections formed at one of the displacing member and the housing, and one or more recesses formed at the other one of the displacing member and the housing for engagement with the projections;
wherein there are at least a plurality of the recesses or a plurality of the projections; and
wherein the plurality of projections or recesses are aligned in a rotational direction of the displacing member.

25. The lancing depth-adjustable lancing apparatus according to claim 15, wherein the cover is attachable to and detachable from the displacing member, and a position of the displacing member relative to the housing is adjusted by applying a load to the displacing member with the cover detached.

26. The lancing depth-adjustable lancing apparatus according to claim 25, wherein the cover covers at least part of the displacing member when attached to the displacing member; and
wherein the part of the displacing member to be covered by the cover is provided with a scale used for adjusting the lancing depth.

27. The lancing depth-adjustable lancing apparatus according to claim 15, wherein the cover is made transparent or translucent so that inside can be observed.

28. The lancing depth-adjustable lancing apparatus according to claim 17, wherein the cover is rotatable independently from the displacing member.

29. A lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin;
wherein the lancing depth adjustment mechanism comprises a cover which is attachable to and detachable from the housing, and a displacing member which is movable relative to the housing in the lancing direction or in a retreating direction which is opposite from the lancing direction independently from the cover.

30. The lancing depth-adjustable lancing apparatus according to claim 29, wherein displacing member is provided with a stepped portion for restricting the movement of the lancing element in the lancing direction when the lancing element moves in the lancing direction.

31. The lancing depth-adjustable lancing apparatus according to claim 29, wherein the displacing member moves relative to the housing in the lancing or the retreating direction when rotated.

32. The lancing depth-adjustable lancing apparatus according to claim 31, wherein the housing includes a main body, and a guide member non-rotatably fixed to the main body; and
wherein the displacing member is rotatably and threadingly engaged with the guide member.

33. The lancing depth-adjustable lancing apparatus according to claim 29, wherein a position of the displacing member relative to the housing is adjusted by applying a load to the displacing member with the cover detached.

34. A lancing depth-adjustable lancing apparatus for moving a lancing element held in a housing in a lancing direction to lance skin with the lancing element, the lancing apparatus comprising a lancing depth adjustment mechanism for adjusting lancing depth of the lancing element in the skin;
wherein the lancing depth adjustment mechanism utilizes a cam mechanism and comprises a displacing member which is movable relative to the housing in the lancing direction or in a retreating direction which is opposite from the lancing direction, and a cover which is movable with the displacing member in the lancing and the retreating directions.

35. The lancing depth-adjustable lancing apparatus according to claim 34, wherein the housing is provided with a stepped portion for restricting the movement of the lancing element in the lancing direction when the lancing element moves in the lancing direction.

36. The lancing depth-adjustable lancing apparatus according to claim 34, wherein the cam mechanism comprises an operation member which is used to move the displacing member and which includes a shaft portion, and a first and a second groove portions respectively provided at the housing and the displacing member for allowing movement of the shaft portion; and
wherein a position of the displacing member relative to the housing is changed by moving the shaft portion in the first and the second groove portions.

37. The lancing depth-adjustable lancing apparatus according to claim 36, wherein one of the first and the second groove portions extends in a direction perpendicular to the lancing and the retreating directions, whereas the other one of the first and the second groove portions extends in a direction inclined with respect to the lancing and the retreating directions.

38. The lancing depth-adjustable lancing apparatus according to claim 34, wherein the cover is attachable to and detachable from the displacing member.
